(19) Europäisches Patentamt
European Patent Office
Office européen des brevets

(11) Veröffentlichungsnummer : **0 052 769**
**B1**

(12) # EUROPÄISCHE PATENTSCHRIFT

(45) Veröffentlichungstag der Patentschrift : **03.04.85**

(51) Int. Cl.⁴ : **G 01 N 21/07**, G 01 N 33/50

(21) Anmeldenummer : **81108750.1**

(22) Anmeldetag : **22.10.81**

(54) **Verfahren zur Durchführung analytischer Bestimmungen und hierfür geeignetes Rotoreinsatzelement.**

(30) Priorität : **25.11.80 DE 3044385**

(43) Veröffentlichungstag der Anmeldung : **02.06.82 Patentblatt 82/22**

(45) Bekanntmachung des Hinweises auf die Patenterteilung : **03.04.85 Patentblatt 85/14**

(84) Benannte Vertragsstaaten : **AT BE CH DE FR GB IT LI LU NL SE**

(56) Entgegenhaltungen :
**DE-A- 2 520 714**
**DE-A- 2 529 245**
**FR-A- 2 400 700**
**FR-A- 2 409 514**
**GB-A- 2 017 910**
**US-A- 4 035 156**
**Die Akte enthält technische Angaben, die nach dem Eingang der Anmeldung eingereicht wurden und die nicht in dieser Patentschrift enthalten sind.**

(73) Patentinhaber : **Boehringer Mannheim GmbH**
**Sandhoferstrasse 116**
**D-6800 Mannheim 31 (DE)**

(72) Erfinder : **Klose, Sigmar, Dr.**
**Breitenloh 7**
**D-8131 Berg 2 (DE)**
Erfinder : **Stähler, Fritz, Dr.**
**Heimgartenstrasse 4**
**D-8132 Tutzing (DE)**
Erfinder : **Lange, Hans**
**Danzigerstrasse 3**
**D-6840 Lampertheim (DE)**
Erfinder : **Kleemann, Wolfgang, Dr.**
**Gröberweg 3**
**D-8132 Tutzing (DE)**

(74) Vertreter : **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel**
**Postfach 860820**
**D-8000 München 86 (DE)**

**EP 0 052 769 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Durchführung analytischer Bestimmungen unter der Einwirkung einer Zentrifugalkraft nach der Zentrifugalanalysen-Methode und ein zur Durchführung dieses Verfahrens bestimmtes Rotoreinsatzelement.

Mit der zunehmenden Bedeutung der klinisch-chemischen Analyse, insbesondere von Blut- und Serumbestandteilen als Basis für die medizinische Diagnose wurden Verfahren und Vorrichtungen entwickelt, welche den zur Durchführung derartiger Analysen erforderlichen Zeit- und Arbeitsaufwand herabsetzen, um die drastisch gestiegene Zahl derartiger Analysen bewältigen zu können. Ein wesentlicher Schritt vorwärts war die Entwicklung von Analysenautomaten. Bei den stets steigenden Anforderungen an Kapazität, Schnelligkeit und Variabilität derartiger Analysenautomaten stiegen auch die Kosten für diese Apparaturen sehr stark an, so daß die modernen Großautomaten nur noch von besonders leistungsfähigen Kliniken und Instituten beschafft werden konnten. Einen wesentlichen Fortschritt in Richtung der Herabsetzung des Bauaufwandes für solche Analysenautomaten brachte das erstmals von Norman G. Anderson in Science, Vol. 166, 317-324 (1969) beschriebene Prinzip der Zentrifugalanalyse. Dieses Prinzip beruht darauf, das Mischen von Probe und Reagenz unter dem Einfluß der Schwerkraft in einem Zentrifugenrotor erfolgt, der nahe am äußeren Rande eine Anzahl von Meßkammern aufweist, die das Reaktionsergebnis zu messen gestatteten, während der Rotor noch in Bewegung ist, die Zentrifugalkraft also noch einwirkt. Insbesondere war es auf diese Weise möglich, innerhalb kürzester Zeit eine erhebliche Anzahl von gleichen Untersuchungen parallel nebeneinander her ablaufen und ausmessen zu lassen. Im Zuge der Weiterentwicklung dieses Analysenprinzips wurde vor allem die Form der Rotoren immer komplizierter gestaltet, um den verschiedenen Arbeitsgängen, wie Mischen von Reagenzien, Inkubation, Ablaufenlassen von Reaktionen und dergleichen in immer besserem Maße nachkommen zu können. Dies führte schließlich zur Entwicklung von sehr kompliziert und aufwendig gestalteten Zentrifugalanalysen-Rotoren, die nicht nur zunehmend teurer, sondern mit zunehmend komplizierterer Gestaltung auch größer wurden. Der Vorteil des Zentrifugalanalysen-Systems hinsichtlich einfacherer und damit auch billigerer Bauweise wurde damit zum Teil wieder aufgegeben.

Es besteht daher ein Bedarf an einem geschlossenen, kompakten Analysensystem, welches zur Bedienung kein hochqualifiziertes technisches Personal erfordert, rasch arbeitet, ein Analysenergebnis liefert, das in seiner Qualität von der Bedienung des Geräts und dem Geschick oder Aufmerksamkeit des Bedienungspersonals unabhängig ist uns es gleichzeitig gestattet, alle in Betracht kommenden Analysen ohne Austausch des Rotors durchzuführen. Insbesondere bestand ein Ziel der Erfindung in der Schaffung eines derartigen Analysensystems, welches es gestattet, gleichzeitig ganz verschiedene Bestimmungen nebeneinander durchführen zu können, um beispielsweise Profilanalysen in einem einzigen Arbeitsgang zu erstellen.

Gelöst wird diese Aufgabe durch das im Anspruch 1 definierte Verfahren zur Durchführung analytischer Bestimmungen und durch das Einsatzelement für einen Zentrifugalanalysen-Rotor nach Anspruch 7.

Das erfindungsgemäße Verfahren zur Durchführung analytischer Bestimmungen durch Mischen und Inkubieren einer Probelösung mit wenigstens einer Reagenzlösung und optische Messung eines Parameters im inkubierten Reaktionslösungsgemisch, wobei Mischen, Inkubieren und Messen während der Einwirkung einer Zentrifugalkraft durchgeführt werden, ist dadurch gekennzeichnet, daß man die Probelösung unter der Einwirkung der Zentrifugalkraft

a) mit einem löslichen Trockenreagenz zusammenbringt unter wenigstens teilweiser Auflösung desselben,

b) das Gemisch bzw. die Lösung durch eine Vielzahl sehr kleiner, miteinander in Verbindung stehender Hohlräume schleudert,

wobei man Zentrifugalkraft und Strömungswiderstand der kleinen Hohlräume so aufeinander abstimmt, daß eine vollständige Auflösung und Mischung der Reaktionspartner und gegebenenfalls Inkubation eintritt, bevor die Reaktionslösung aus den kleinen Hohlräumen in eine Meßkammer austritt, in der man die Messung vornimmt.

Das erfindungsgemäße Verfahren gestattet es, auf die bisherige komplizierte Ausbildung von Zentrifugalanalysator-Rotoren zu verzichten und durch einen ganz einfach ausgebildeten Rotor zu ersetzen, der zur Aufnahme von austauschbaren Einsatzelementen eingerichtet ist, welche trotz ihres einfachen mechanischen Aufbaus sämtliche Manipulationen überflüssig machen und lediglich noch die Zugabe der Probelösung erfordern. Die erfindungsgemäß vorgesehene Vielzahl kleiner Hohlräume setzt der unter dem Einfluß der Zentrifugalkraft nach außen in die Meßkammer strömenden Flüssigkeit einen solchen Strömungswiderstand entgegen und bewirkt gleichzeitig eine so vollständige Durchmischung von Probelösung und dem sich in der Probelösung auflösenden Reagenz, daß besondere räumliche Gestaltungen von Mischkammern, Verbindungskanälen und dergleichen überflüssig werden und allein durch die Wahl der Abmessungen der kleinen Hohlräume jede in Betracht kommende Strömungsgeschwindigkeit und Mischintensität bei gegebener Zentrifugalkraft erzielt werden kann. Die hierfür erforderliche Vielzahl von kleinen Hohlräumen läßt sich auf einfachste Weise durch Verwendung eines

netzförmigen Elements, wie eines geflochtenen Netzes, eines Papierstreifens, Vlieses oder dergleichen, oder eines offenzelligen Schaumstoffs oder einer strukturierten Oberfläche erreichen. Die darin enthaltenen Poren und Vertiefungen bilden die kleinen, miteinander in Verbindung stehenden Hohlräume, deren Anwendung ein wesentliches Merkmal der Erfindung ist.

Die Hohlraumgröße entspricht daher der Größe der offenen Räume in derartigen netzförmigen Elementen und wird normalerweise etwa 2 mm, vorzugsweise 1 mm, nicht übersteigen. Die Untergrenze wird durch die Passierbarkeit für die Lösung unter Einwirkung der Zentrifugalkraft bestimmt.

Statt eines netzförmigen Elements eignet sich beispielsweise auch ein offenzelliger Schaumstoff oder eine strukturierte Oberfläche, die mit einer zweiten glatten oder ebenfalls strukturierten Oberfläche abgedeckt ist. Als strukturierte Oberfläche kommen sowohl aufgerauhte Oberflächen in Betracht als auch solche, welche mit einer Vielzahl kleiner taschenartiger Vertiefungen versehen sind. Die Verbindung zwischen den einzelnen sehr kleinen Hohlräumen wird in diesem Falle erhalten, indem die zweite gegenüberliegende Oberfläche nicht dicht anliegt, sondern einen geringen, für den Durchtritt von Flüssigkeit unter dem Einfluß der Schwerkraft ausreichenden Abstand aufweist. Liegen dabei zwei strukturierte Oberflächen aneinander, so kann die Strukturierung auf beiden Seiten unterschiedlich sein, so daß je nach Drehrichtung des Rotors unterschiedliche Effekte bewirkt werden können.

Ebenfalls können in Fließrichtung der Flüssigkeit, also in Richtung auf die Meßkammer, Elemente mit unterschiedlicher Größe der kleinen Hohlräume und dementsprechend unterschiedlichem Strömungswiderstand bei gegebener Zentrifugalkraft angeordnet werden. Auf diese Weise ist es möglich, nach Belieben die Strömungsgeschwindigkeit in einzelnen Abschnitten des Strömungswegs von der Probenaufgabe zur Meßkammer zu erhöhen oder zu verringern.

Ein wesentlicher Vorteil der Erfindung ist die Möglichkeit, mehrere verschiedene Trockenreagenzien vorzusehen, mit denen man die Probelösung unter dem Einfluß der Zentrifugalkraft zusammenbringt. Das erfindungsgemäße Verfahren eignet sich daher auch zur Durchführung mehrstufiger analytischer Bestimmungen, bei denen nacheinander verschiedene Reaktionen ablaufen gelassen werden. Desgleichen ist es möglich, verschiedene, miteinander nicht verträgliche Bestandteile eines Reagenz räumlich getrennt anzuordnen, und zwar sowohl innerhalb der kleinen Hohlräume als auch außerhalb derselben.

Gemäß einer ersten Ausführungsform des erfindungsgemäßen Verfahrens läßt man die Probelösung, die mehr oder minder vorverdünnt eingesetzt werden kann, durch eine mit den sehr kleinen Hohlräumen besetzte Strecke strömen, bei der die mittlere Hohlraumgröße und der mittlere Strömungswiderstand überall den gleichen Wert aufweist. Diese Ausführungsform eignet sich besonders bei Verfahren, die einstufig ablaufen. Gewünschtenfalls kann als Bremsstrecke noch eine Strecke mit erhöhtem Strömungswiderstand nachgeschaltet werden, z. B. um die Inkubationszeit bis zum Eintritt in die Meßkammer zu erhöhen. Dies ist im folgenden Absatz näher erläutert.

Eine weitere bevorzugte Ausführungsform des erfindungsgemäßen Verfahrens ermöglicht die Anwendung von zwei Inkubationsstufen unter Verwendung von zwei verschiedenen Reagenzien. Hierbei läßt man die Probeflüssigkeit zuerst durch ein erstes, eine Vielzahl sehr kleiner, miteinander in Verbindung stehender Hohlräume aufweisendes Element strömen, welches ein erstes Trockenreagenz enthält. In Strömungsrichtung dahinter angeordnet findet sich ein zweites Element mit einer Vielzahl sehr kleiner, miteinander in Verbindung stehender Hohlräume mit größerem Strömungswiderstand als das erste Element. Dieses zweite Element kann beispielsweise ein netzförmiger Körper mit dichterer Packung der Fäden als im ersten Element oder ein Schaumstoff mit kleineren Poren sein. Dieses zweite Element hat infolgedessen einen größeren Strömungswiderstand und bremst die Flüssigkeit ab, so daß die erste Reaktion zwischen der Probelösung und dem ersten Reagenz ablaufen kann. Hinter diesem zweiten Element ist dann ein drittes Element mit einer Vielzahl sehr kleiner, miteinander in Verbindung stehender Hohlräume angeordnet, welches wiederum einen geringeren Strömungswiderstand besitzt als das zweite Element und welches ein zweites, vom ersten verschiedenes Reagenz enthält. Die Probelösung durchströmt also bei dieser Ausführungsform der Erfindung zwei Inkubationsstufen, zwischen denen sich eine Bremsstrecke befindet. Eine weitere Bremsstrecke kann nachgeschaltet werden.

Eine weitere bevorzugte Ausführungsform der Erfindung entspricht der vorstehend aufgeführten Methode mit zwei Inkubationsstufen und einer dazwischengeschalteten Abbremsung der Flüssigkeit, weist jedoch zwischen dem zweiten und dem dritten Element noch ein zusätzliches Element auf, in dem eine Trennung durchgeführt werden kann. In diesem zusätzlichen, vierten Element weisen die sehr kleinen Hohlräume entweder eine reaktive Oberfläche auf oder sind so ausgebildet, daß sie eine Molekularsiebwirkung entfalten.

Im Falle einer reaktiven Oberfläche weist diese entweder als Ionenaustauscher wirkende Gruppen auf, enthält Gruppen, die eine Affinitätschromatographie-Wirkung entfalten oder enthält enzymatisch oder immunologisch aktive Körper kovalent oder in anderer Weise gebunden. Die Fixierung von Substanzen der oben genannten Art, also von enzymatisch aktiven, immunologisch aktiven oder für eine Affinitätschromatographie geeigneten Substanzen an Oberflächen von Festkörpern ist dem Fachmann bekannt und braucht hier nicht näher erläutert zu werden. Wenn das vierte Element beispielsweise ein netzförmiger Körper ist, welcher aus Cellulose oder Polyamidfasern besteht, so lassen sich für die Aktivierung der Oberfläche beispielsweise die in DE-A-27 08 018 und 24 38 436 beschriebenen Verfahren anwenden. Gleiches gilt bei affinitätschromatographisch wirksamen Substanzen, die an die Faseroberflächen fixiert werden. Bei Oberflächen mit Ionenaustauscherfunktion kann das vierte Element aus einem der bekannten Ionenaustauschermaterialien bestehen, beispielsweise auf Basis von sulfonierten oder amidierten Polystyrolharzen, Cellulosefasern oder vernetzten Dextrangelen. Das vierte Element mit chemisch reaktiver Oberfläche kann auch aus einer dichten Packung sehr kleiner Körnchen mit reaktiver Oberfläche bestehen, die aus den dem Fachmann hierfür bekannten Substanzen aufgebaut sein können. Geeignete Materialien sind Glas, Metalle, Kunststoffe, Keramikkörnchen und ähnliche, dem Fachmann als Träger für chromatographisch oder biologisch aktive Substanzen bekannte Materialien.

Das Trockenreagenz ist wie erwähnt vorzugsweise innerhalb der sehr kleinen, von der Probeflüssigkeit zu durchströmenden Hohlräume angeordnet. Es ist jedoch auch möglich, das Reagenz, beispielsweise in granulierter oder tablettierter Form vor den sehr kleinen Hohlräumen oder in einer Unterbrechung derselben anzuordnen. Indem man die Strömungsgeschwindigkeit durch geeignete Wahl von Zentrifugalkraft und Größe der kleinen Hohlräume entsprechend gering wählt, kann die Kontaktzeit zwischen dem festen Reagenz und der Probelösung in weiten Grenzen festgelegt werden, so daß ausreichend Zeit zur vollständigen Auflösung des Reagenz in der Probelösung zur Verfügung steht. Je nach der Natur des verwendeten Reagenz kann es jedoch ausreichen, wenn nur ein Teil desselben aufgelöst wird. In diesen Fällen wird vorzugsweise das Reagenz in einem Überschuß über die zur Umsetzung mit der Probelösung erforderlichen Menge eingesetzt. Ebenso kann das Reagenz auch schwerlösliche oder unlösliche Partikel enthalten, die sich nur verzögert oder gar nicht in der Probelösung auflösen. Derartige unlösliche Partikel sollten eine Teilchengröße aufweisen, die wesentlich unter der Größe der kleinen Hohlräume und ihrer Verbindungsöffnungen liegt, um ein einwandfreies Passieren der kleinen Hohlräume zu ermöglichen. Es ist jedoch auch möglich, auf einem Teil der Strecke die sehr kleinen Hohlräume so zu gestalten, daß sie eine Siebfunktion auf derartige unlösliche Partikel ausüben.

Die beschriebene Arbeitsweise unter Anordnung von Elutions- und Mischstrecken, Brems- und Inkubationsstrecken und reaktiven Oberfläche-Strecken kann natürlich auch durch Wiederholung dieser Verfahrensabschnitte ergänzt werden, beispielsweise indem die drei vorstehend erläuterten Arbeitsweisen beliebig kombiniert werden.

Die Messung des Reaktionsergebnisses kann nach den hierfür üblichen Methoden erfolgen, beispielsweise also optisch unter Bestimmung des Reaktionsendpunkts oder Aufnahme einer Kinetik. Ebenso können Leitfähigkeitsmessungen vorgenommen werden.

Ein weiterer Gegenstand der Erfindung ist ein Einsatzelement für einen Zentrifugalanalysenrotor, welches dadurch gekennzeichnet ist, daß es wenigstens ein Analysenreagenz in getrockneter Form enthält und eine Vielzahl sehr kleiner, miteinander in Verbindung stehender Hohlräume aufweist, welche eine Probeaufgabekammer und eine Meßkammer miteinander verbinden.

Das erfindungsgemäße Einsatzelement ermöglicht eine besonders einfache Durchführung des erfindungsgemäßen Verfahrens und läßt die durch das Verfahren ermöglichte, gegenüber bisher bekannten Zentrifugalanalysenrotoren erreichte entscheidende konstruktive Vereinfachung voll zum Tragen kommen. Das erfindungsgemäße Einsatzelement wird zusammen mit einer Rotoreinheit für einen Zentrifugalanalysator angewendet, welcher eine mit einem Antrieb verbundene Rotorbasis und einen im Betrieb mit der Rotorbasis verbundenen Rotorkopf aufweist, in welchem Kammern für die Aufnahme einer Probenflüssigkeit und radial auswärts von den jeweils zugeordneten Probekammern Meßkrammern für die Messung von für den Nachweis von Bestandteilen der Probe charakteristischen Parametern sowie Fluidkanäle zur Verbindung der Probekammern und der Meßkammern einschließt, wobei die Rotoreinheit dadurch ausgezeichnet ist, daß der Rotorkopf eine Mehrzahl verschiedener erfindungsgemäßer Einsatzelemente einschließt, die auswechselbar und mit der Rotorbasis an verschiedene Stellen wahlweise im Betrieb positionsstabil verbindbar sind. Eine derartige Rotoreinheit ist in DE-A-3 044 372.

Das erfindungsgemäße Einsatzelement kann allein aus einem netzförmigen Element, welches eine Vielzahl sehr kleiner, miteinander in Verbindung stehender Hohlräume definiert, bestehen. Beispiele für derartige netzförmige Elemente sind ein geflochtenes Netz, Vlies, Papier, offenzellige Schaumstoffe, dichtgepackte kleine Körper und dergleichen. Wenn das Einsatzelement alleine aus einem derartigen Element mit den sehr kleinen, miteinander in Verbindung stehenden Hohlräumen besteht, so ist das Analysenreagenz in getrockneter Form in den Hohlräumen enthalten. Im einfachsten Fall kann also ein derartiges Einsatzelement nur aus einem Stück Vlies oder Papier bestehen, welches mit dem Analysenreagenz imprägniert ist. Ein derartiges Einsatzelement kann beispielsweise so angewendet werden, daß ein Zentrifugalanalysenrotorkörper, welcher eine Anzahl von kreisförmig angeordneten Probeaufgabekammern und eine Anzahl von diesen zugeordneten, radial auswärts davon angeordneten Meßkammern, die miteinander durch radiale Schlitze verbunden sind, vorgesehen wird, in diese Schlitze das erfindungsgemäße Einsatzelement dicht passend eingesetzt wird, die Probelösung in die Probeaufgabekammer eingebracht und nach Verschließen durch eine Rotordeckelplatte oder dergleichen eine vorbestimmte Zentrifugalkraft erzeugt wird unter deren Einfluß die Probeflüssigkeit durch das Vlies oder Papier nach außen geschleudert wird. Hierbei durchströmt die Flüssigkeit die vielen sehr kleinen, miteinander in Verbindung stehenden Hohlräume, löst das Trockenreagenz auf, bewirkt eine vollständige Durchmischung der Reaktionspartner und eine Inkubation durch die beim Übergang von einem der sehr kleinen Hohlräume zum nächsten erforderliche Umlenkung der Strömungsrichtung und gelangt schließlich in die Meßkammer, in der man in an sich bekannter Weise die Messung durchführt.

In einer weiteren Ausführungsform des erfindungsgemäßen Einsatzelements kann dieses jedoch außer dem die Vielzahl sehr kleiner, miteinander in Verbindung stehender Hohlräume aufweisenden Element auch eine Probeaufnahmekammer oder/und Meßkammer aufweisen. Eine derartige Ausführungsform des erfindungsgemäßen Einsatzelements kann beispielsweise aus dem die sehr kleinen Hohlräume aufweisenden Element in Form eines länglichen Körpers bestehen, der mit einer Folie, die beispielsweise aus Kunststoff besteht, nach außen abgedichtet ist, wobei an wenigstens einer Schmalseite des länglichen Körpers die Folie eine Schlaufe bildet, die eine Meßkammer oder/und Probeaufgabekammer definiert. Ein derartiges Einsatzelement läßt sich daher beispielsweise auf äußerst einfache Weise herstellen, indem man einen mit dem trockenen Analysenreagenz imprägnierten rechteckigen Papier- oder Vliesstreifen auf eine Kunststoffolie legt, die zu beiden Seiten geringfügig, z. B. 0,5 bis 1 mm, übersteht. Die Folie wird an einem Ende umgeschlagen und auf die andere Seite des Streifens gelegt, derart, daß am umgelegten Ende eine kleine Schlaufe gebildet wird. Durch Versiegeln der überstehenden Kanten der Folie entsteht das fertige Einsatzelement. Anstelle einer Folie können natürlich auch entsprechend geformte Körper aus Kunststoff oder ähnlichem formbaren Material verwendet werden. Das erfindungsgemäße Einsatzelement kann mehrere räumlich voneinander getrennt vorliegende, in der Regel verschiedene, Analysenreagenzien enthalten. Die Analysenreagenzien können wie bereits erwähnt, innerhalb der sehr kleinen Hohlräume vorliegen. Beispielsweise gibt man eine bestimmte Menge einer Lösung des Analysenreagenz auf eine Stelle des die kleinen Hohlräume aufweisenden Elements, wie eines Vlies- oder Papierstreifens und trocknet ihn dort, beispielsweise durch Lyophilisierung oder andere Trocknungsmethoden. Alternativ kann das Analysenreagenz im erfindungsgemäßen Einsatzelement auch als geformter Körper, beispielsweise in Form eines Granulats, in tablettierter Form oder dergleichen vorliegen und dann in der Regel außerhalb der sehr kleinen Hohlräume angeordnet sein.

Das erfindungsgemaße Einsatzelement kann auf wenigstens einem Teil der Oberfläche der kleinen Hohlräume reaktiv ausgebildet sein. Wie oben bereits erwähnt, kann hierbei der netzförmige Körper aus Fasern oder Fäden bestehen, an deren Oberfläche reaktive Substanzen, beispielsweise enzymatisch oder immunologisch reaktive Substanzen fixiert sind. Auf die obigen Ausführungen im Rahmen der Erläuterung des erfindungsgemäßen Verfahrens wird verwiesen.

Beim erfindungsgemäßen Einsatzelement können mehrere Elemente oder Körper, welche eine Vielzahl sehr kleiner, miteinander in Verbindung stehender Hohlräume aufweisen, nebeneinander oder vorzugsweise hintereinander angeordnet werden, so daß es möglich ist, durch unterschiedliche Größe der kleinen Hohlräume unterschiedliche Strömungswiderstände und damit auch Strömungsgeschwindigkeiten einzustellen, ohne daß eine Variation der Zentrifugalkraft durch Drehzahländerung erforderlich wäre. Im Fall eines netzförmigen Elements wird beispielsweise der Strömungswiderstand durch die Dicke der Fasern und die Art ihrer Verbindung bedingt. Bei abnehmendem Faserdurchmesser werden die mittleren Durchmesser der kleinen Hohlräume geringer und der Strömungswiderstand steigt entsprechend an. Durch die Art der Verbindung der einzelnen Fasern wird die Größe der Durchlässe zwischen den sehr kleinen Hohlräumen ebenfalls beeinflußt und durch Ausnutzung dieses Effekts läßt sich ebenfalls der Strömungswiderstand auf eine bestimmte gewünschte Größe einstellen. Auf diese Weise ist es möglich, innerhalb des Einsatzelements Strecken mit größerem und geringerem Strömungswiderstand vorzusehen und damit eine Beschleunigung oder Abbremsung der Strömungsgeschwindigkeit der Reaktionslösung zu bewirken.

Ein bevorzugtes Einsatzelement ist dadurch gekennzeichnet, daß zwischen Probeaufgabekammer und Meßkammer Abschnitte mit unterschiedlicher mittlerer Hohlraumgröße angeordnet sind, welche für eine durchströmende Flüssigkeit unterschiedlichen Strömungswiderstand aufweisen.

Ein weiteres bevorzugtes Einsatzelement ist gekennzeichnet durch ein erstes Element, welches eine Vielzahl sehr kleiner, miteinander in Verbindung stehender Hohlräume aufweist, und ein erstes Reagenz enthält, ein in Strömungsrichtung dahinter angeordnetes zweites Element mit einer Vielzahl sehr kleiner, miteinander in Verbindung stehender Hohlräume mit größerem Strömungswiderstand als das erste Element und ein in Strömungsrichtung dahinter angeordnetes drittes Element, welches eine Vielzahl sehr kleiner, miteinander in Verbindung stehender Hohlräume aufweist, die einen geringeren Strömungswiderstand besitzen als die Hohlräume des zweiten Elements, und ein zweites Reagenz enthält.

Ein weiteres bevorzugtes Einsatzelement ist dadurch gekennzeichnet, daß es zwischen dem zweiten und dritten Element ein weiteres Element mit einer Vielzahl sehr kleiner, miteinander in Verbindung stehender Hohlräume aufweist, welche eine reaktive Oberfläche aufweisen.

Ein derartiges erfindungsgemäßes Einsatzelement enthält vorzugsweise auch Elemente, die an der Oberfläche enzymatisch oder immunologisch aktive Substanzen gebunden aufweisen.

Ein weiteres bevorzugtes Einsatzelement gemäß der Erfindung ist dadurch gekennzeichnet, daß es Elemente enthält, deren Oberfläche reaktive Gruppen mit Ionenaustauschereigenschaften aufweist.

Das erfindungsgemäße Verfahren zur Durchführung analytischer Bestimmung und das hierfür geeignete Rotoreinsatzelement werden nachstehend in Verbindung mit der beigefügten Zeichnung näher beschrieben. In dieser stellen dar:

Figur 1 eine Außenansicht eines Zentrifugalanalysenautomaten, der für die Anwendung der Erfindung geeignet ist,

Figur 2 einen Schnitt durch die Ebene eines Zentrifugalanalysatorrotors,

Figur 3 einen Querschnitt durch den Rotor von Fig. 2,

Figur 4 eine Seitenansicht eines erfindungsgemäßen Einsatzelements,
Figur 5 einen Längsschnitt durch das Einsatzelement von Fig. 4,
Figur 6 eine schematische Ansicht des Einsatzelements der Fig. 4 und 5 auf einem Rotor,
Figuren 7a, 7b und 7c schematische Darstellungen von strukturierten Oberflächen mit sehr kleinen, miteinander in Verbindung stehenden Hohlräumen,
Figuren 8, 9 und 10 graphische Darstellungen der nach den Beispielen erhaltenen Analysenresultate.

Fig. 1 zeigt eine Außenansicht eines Zentrifugalanalysengeräts, welches für die Erfindung geeignet ist. Ein Einzelteile des Geräts aufnehmendes Gehäuse 1 enthält einen Zentrifugenrotor 2. Die gezeigte Rotorausführungsform entspricht dem in den Fig. 2 und 3 näher beschriebenen Rotor. Der Rotor wird durch nicht gezeigte Antriebselemente in der bei derartigen Analysengeräten bzw. Zentrifugen üblichen Weise befestigt und angetrieben. Zwei Bedienungstastenfelder 3a und 3b ermöglichen die Vornahme der erforderlichen Manipulationen und Eingriffe in die Steuerung. Ein Bildschirm 4 ermöglicht die optische Wiedergabe von Analysenresultaten und die Abrufung gespeicherter Informationen. 5 und 6 sind Eingabe- bzw. Ausgabestationen für Magnetkarten, Holerithkarten oder ähnliche Speichereinrichtungen für dem Gerät zuzuführende Informationen.

In den Fig. 2 und 3 ist eine für die Erfindung geeignete Rotorausführung dargestellt, die im Prinzip aus drei aufeinander befestigten runden Scheiben besteht, von denen Fig. 2 eine Aufsicht der mittleren Scheibe und Fig. 3 einen Schnitt durch den Rotor auf der Linie III-III zeigt.

Der Rotor 21 besteht aus einer schwarzen Mittelscheibe 22 als Mittelteil und zwei transparenten Kunststoffplatten 23 und 24, die als Boden und Deckel dienen. In der beispielsweise schwarz gefärbten Mittelscheibe 22 von beispielsweise 6 mm Höhe und 33 mm Durchmesser sind auf einem Radius von z. B. 28 mm Durchmesser fünf gleichmäßig verteilte Löcher 25 von beispielsweise 1,7 mm Durchmesser angeordnet, die als Meßkammer, speziell als Küvetten, dienen. Zum Mittelpunkt der Scheibe 22 hin schließt sich an jede Meßkammer 25 eine Vorkammer 25a von z. B. 2 mm × 5 mm an. Danach folgt ein schlitzartiger Kanal 26 von z. B. 6 mm Länge und 1 mm Breite. Der Kanal 26 ist auf halber Höhe von der Vorkammer durch eine Barriere 27 getrennt. Die Barriere 27 verhindert ein Zurückfließen der Lösung während der Mischschritte. Vor dem Kanal 26 nach innen zu liegt die Probenkammer 28 mit einem Durchmesser von z. B. 3 mm. Diese setzt sich auch nach unten in das Bodenteil 24 fort und ist so gestaltet, daß die bei der Beschleunigung des Rotors auftretenden Trägheitskräfte die verdünnte Probe auf den Elutionskanal 26 zutreiben. Die Vertiefungen im Boden 24 sind beispielsweise so bemessen, daß 20 µl nicht ganz bis an den Elutionskanal anstehen. Das zur Füllung der Küvette 25 benötigte Volumen einschließlich der Verluste durch Retention auf dem Einsatzelement 29 beträgt beispielsweise 18 µl. Das Einsatzelement 29 besteht aus einem mit einem Analysenreagenz in fester Form imprägnierten rechteckigen Papierstreifen, der dicht passend in den schlitzartigen Kanal 26 eingesetzt ist.

Bodenteil 24 und Deckelteil 23 bestehen beispielsweise aus durchsichtigem Plastikmaterial und bilden auch die Fenster für die Küvetten 25. Der Boden 24 wird z. B. durch Ultraschallverschweißung mit dem Mittelteil 22 verbunden. Der Deckel 23 wird nur aufgeschraubt, wobei Silikongummidichtungen 30 um Küvetten 25 und Kanäle 26 herum angeordnet werden können. Im Deckel 23 befinden sich über den Kanälen 26 Aussparungen 31, die verhindern, daß Flüssigkeit durch Kapillarwirkung des Spalts zwischen Mittelkörper 22 und Deckelteil 23 zurückgesaugt werden kann.

Die Fig. 4 und 5 zeigen ein weiteres erfindungsgemäßes Einsatzelement, welches als Wegwerfteil ausgebildet ist und sich zur Verwendung auf einem größeren Rotor eignet. Das Einsatzelement 41 besteht, von links nach rechts betrachtet, aus der Probenkammer 42, einer Anzahl Felder 43 (43, 44, 45, 46, 47, 48, 49), in der Einsatzkörper mit einer Vielzahl miteinander in Verbindung stehender Hohlräume eingesetzt werden können, welche z. B. Reagenz enthalten, den Flüssigkeitsstrom abbremsen können, eine reaktive Oberfläche aufweisen können usw. An die Felder 43 bis 49 folgt eine als Mischzone ausgebildete Strecke 50 und die Meßkammer (Küvette) 51. Die Strecke 50 kann aus zwei dicht aneinanderliegenden strukturierten Oberflächen oder einem Netz bestehen, wie in den Fig. 7a bis 7c näher erläutert. Sind die Felder 43 bis 49 beispielsweise mit Papierstreifen als Elemente mit einer Vielzahl sehr kleiner, miteinander in Verbindung stehender Hohlräume gefüllt, so können diese entsprechend den oben näher erläuterten Verfahrensstufen ausgestaltet sein. Soll beispielsweise mit dem Einsatzelement 41 Glucose bestimmt werden, so befindet sich im Feld 43 ein mit Puffersubstanz imprägniertes Vlies, im Feld 44 ein mit 4-Aminoantipyrin imprägniertes Vlies, in Feld 45 ein mit den Enzymen und der Phenolkomponente imprägniertes Vlies, wobei diese chemischen Substanzen natürlich stets in trockener Form vorliegen.

Im Betrieb wird in die Probeeingabekammer 42 die mit Wasser oder einer anderen geeigneten wäßrigen Lösung verdünnte Probe eingegeben, deren minimale Menge sich aus dem Küvettenvolumen und der unter den gegebenen Umständen hinsichtlich Radius des Rotors und Umdrehungsgeschwindigkeit auf dem Vlies zurückbleibende Menge Lösung ergibt. Nach Beginn der Rotation durchströmt die verdünnte Probe die Vliese in den Feldern 43 bis 49, wobei in den Feldern 46 bis 49 weitere Reagenzien und/oder aktive Oberflächen und/oder Veränderungen der Strömungsgeschwindigkeit vorgesehen werden können. Die Probe löst nacheinander die benötigten Mengen Puffer, 4-Aminoantipyrin, Enzyme und Phenol auf, wobei auch die Reaktion gestartet wird. In den Feldern 46 bis 49 und der Mischstrecke 50, gegebenenfalls alleine in der Mischstrecke 50, wird eine gute Durchmischung

der Lösung erreicht und die schon reagierende Lösung strömt in die Küvette 51, wo sie in der von Zentrifugalanalysatoren bekannten Weise vermessen werden kann.

Das erfindungsgemäße Einsatzelement 41 läßt in den Feldern 46 bis 49 den Einbau von Elementen mit einer Vielzahl sehr kleiner, miteinander in Verbindung stehender Hohlräume zu, die als Bremskörper, Trennsäulen usw. wirksam sind. Bremskörper sind beispielsweise Elemente mit sehr kleinen Poren, die einen Durchtritt der Flüssigkeit beispielsweise erst nach 5 oder mehr Minuten zulassen, so daß in diesen Feldern auch eine Vorinkubation ablaufen gelassen werden kann. Felder mit Elementen, welche chemisch reaktive Oberflächen aufweisen, eignen sich als Trennsäulen. Gleiches gilt für als Molekularsiebe wirksame Einsätze.

Die Anwendung von Einsatzelementen für die Felder 46 bis 49, die als Bremskammern und Trennsäulen dienen, sei nachfolgend am Beispiel der Bestimmung von Tyroxin ($T_4$) näher erläutert. Die in den Fig. 4 und 5 gezeigten Felder weisen dann Einsätze, beispielsweise Papiervliese, auf, die mit folgenden Substanzen imprägniert sind bzw. nachstehende Wirksamkeit aufweisen :

43 Erster Puffer und Detergenz
44 Markiertes Antigen ($T_4$-POD) ; POD = Peroxidase
45 $T_4$-Antikörper (AK)
46 Bremskammer (Inkubationsstrecke)
47 Trennsäule (Antikörper gegen Anti-$T_4$)
48 Zweiter Puffer, Glucoseoxidase
49 Farbentwicklungssubstrat (z. B. Phenol und 4-Aminoantipyrin).
$T4_P = T_4$ aus der Probe

Dabei laufen die Reaktionen nach folgendem Schema ab :

$$T4_P + T4\text{-POD} + AK \rightarrow T4_P \not\neq AK + T4\text{-POD} \not\neq AK + T4_P + T4\text{-POD}$$

Diese Reaktion findet in der Inkubationsstrecke 46 statt. Die Trennsäule 47 enthält Antikörper gegen Anti-T4 gebunden, so daß sie $T4_P \not\neq AK$ und $T4\text{-POD} \not\neq AK$ vollständig absorbiert. $T4_P$ und T4-POD passieren durch die Säule 47. Nachdem die von den Antikörperkomplexen befreite Lösung die Abschnitte 48 und 49 passiert hat und die dortigen Reagenzien gelöst hat, gelangt die auf diese Weise angereicherte Lösung in die Küvette 51, in der die POD-Aktivität des T4-POD aus Phenol + 4-Aminoantipyrin mittels des aus der Probenglucose unter GOD-Einfluß erzeugten $H_2O_2$ einen Farbkomplex bildet, dessen Konzentrationsanstieg bei 500 nm gemessen wird.

Wie aus Fig. 6 ersichtlich, ist ein derartiges Einsatzelement durch hier nicht gezeigte Befestigungsmittel auf einem Rotor so befestigt, daß von oben eine Probeneingabe durch ein geeignetes Dosiergerät und eine Durchstrahlung der Küvette zur Messung des Reaktionsergebnisses ermöglicht wird.

Fig. 7 zeigt schematisch mögliche Anordnungen der sehr kleinen Hohlräume, wobei a und b strukturierte Oberflächen und c ein netzförmiges Element darstellen.

Die erfindungsgemäßen Einsatzelemente können auch gebündelt zu größeren segmentförmigen Einheiten zusammengefaßt vorliegen, so daß sich ein Analysenprofil in einem einzigen Arbeitsgang bestimmen läßt, da jedes Einzelelement einen anderen Parameter der eingegebenen Probe zu bestimmen gestattet. Solche segmentförmigen Einsatzelementbündel eignen sich auch zur gleichzeitigen Messung eines bestimmten Parameters in einer Vielzahl von Proben. In diesem Fall enthält jedes Einsatzelement im Einsatzelementbündel eine eigene Probeneingabekammer. Es ist offensichtlich, daß beliebige Kombinationen zwischen diesen Ausführungsformen möglich sind, so daß gleichzeitig viele gleichartige oder/und unterschiedliche Bestimmungen durchgeführt werden können. Die Einsatzelemente können wiederverwendbar oder, was bevorzugt wird, wegwerfbar ausgebildet sein.

Die Reagenzien können in gemischter Form vorliegen oder als Einzelkomponenten, die beim Durchströmen der Probeflüssigkeit nacheinander aufgelöst und miteinander gemischt werden. In diesem Falle können die verschiedenen, im Einsatzelement angeordneten netzförmigen Elemente aus einzelnen Papierstreifen bestehen, die jeweils eine bestimmte Reagenzmenge imprägniert enthalten. Derartige einzelne Komponenten eines Analysenreagenz enthaltende Träger sind beispielsweise in der DE-A-28 52 994 beschrieben.

Mit dem erfindungsgemäßen Verfahren und Einsatzelement lassen sich im Prinzip alle vorkommenden Bestimmungen von Blut und Serumbestandteilen aber auch andere analytische Bestimmungen durchführen. In der nachstehenden Tabelle 1 ist eine Reihe von Beispielen von Bestimmungsmethoden angegeben, die einen einzigen Inkubationsschritt erfordern (« Typ 1 »), die erfindungsgemäß durchgeführt werden können. Die Tabelle gibt die zu bestimmenden Parameter, die zweckmäßige Verdünnung der Probe, die Dauer der Bestimmung in Sekunden, die anzuwendende Meßtechnik und die Anzahl der benötigten Reagenzsegmente an.

$\not\neq$ bezeichnet den Antigen-Antikörper-Komplex.

Tabelle 1

| Parameter | Verd.-faktor | Meßzeit (gesamt) | Meßtechnik | Reagenz-segmente |
|---|---|---|---|---|
| Glucose | 1oo-2oo | <1oo s | Pseudo-EP | 3 |
| Bilirubin total | 1o | 6o s | EP; polychrom. | 1 |
| Creatinin | 1o | <9o s | Pseudo-EP | 2 |
| Albumin | 2oo | 3o s | EP; polychrom. | 1 |
| Gesamteiweiß | 5o | <9o s | Pseudo-EP | 1 (2) |
| Eisen | 4 | 9o s | EP; polychrom. | 3 |
| Hämoglobin | 25o | 165 s | EP; polychrom | 2 |
| Harnstoff | 1oo | 6o s | Pseudo-EP | 3 |
| Harnsäure | 5o | 12o s | Pseudo-EP | 3 |
| Triglyceride | 5o | 15o s | Pseudo-EP | 3 |
| Cholesterin | 1oo-2oo | 15o s | Pseudo-EP | 3 |
| Chlorid | 5o | <6o s | EP; polychrom. | 1 |
| Calcium | 5o | <6o s | EP; polychrom. | 2 |
| Phosphat | 5o | 1oo s | Pseudo-EP | 2 |
| γ-GT | 1o | 9o s | Kinetik | 2 |
| AP | 5o | 12o s | Kinetik | 3 (4) |
| GOT | 1o | 9o s | Kinetik | 3 |
| GPT | 1o | 9o s | Kinetik | 3 |
| LDH | 1oo | 6os | Kinetik | 2 |

EP = Endpunktbestimmung

Pseudo-EP = Verfahren, bei dem mit Hilfe eines bekannten mathematischen Verfahrens Anfangs- und Endextinktion berechnet werden.

In analoger Weise lassen sich beispielsweise auch Lactat und Ammoniak, Lipase, Amylase und Creatinkinase bestimmen.

In der nachfolgenden Tabelle 2 sind weitere erfindungsgemäß bestimmbare Parameter angegeben, die mehr als einen Inkubationsschritt erfordern und beispielsweise die Erstellung eines Schilddrüsenprofils, die Bestimmung spezifischer Proteine, die Bestimmung von Gerinnungsaparametern und von Wirkstoffen ermöglichen.

(Siehe Tabelle 2 Seite 9 ff.)

Tabelle 2

| Parameter | Verdünnungsfaktor | Meßzeit | Meßtechnik | Reaktions-/Reagenzsegmente |
|---|---|---|---|---|
| Lipase | 25 | 18o s | Kinetik | 2 |
| Amylase | 1oo | 18o s | Kinetik | 2 |
| Ccreatinkinase | 1o | 18os | Kinetik | 3 |
| **Schilddrüsenprofil:** | | | | |
| $T_4$ | | | Analog T4[1)] | 7 |
| Trijodthyronin | | | Analog T4 | 7 |
| Thyroxin-bindendes Globulin | | | " | 7 |
| Thyreotropin | | | " | 7 |
| **Enzyme:** | | | | |
| Saure Phosphatase | | | " | |
| **Gerinnung:** | | | | |
| Opt. Quick | | 36o s | Kinetik | 3 |
| Heparin | | 36o s | Kinetik | 3 |
| Plasminogen | | | " | 3 |
| Antiplasmin | | | " | 3 |
| Prothrombin | | | " | 3 |
| Antitrypsin | | | Kinetik | 3 |
| **Drogen:** | | | | |
| Digoxin | | | Analog T4 | 7 |
| Digitoxin | | | " | 7 |
| Phenytoin | | | " | 7 |
| Phenobarbital | | | " | 7 |
| Primidon | | | " | 7 |
| Carbamazepin | | | " | 7 |
| Gentamycin | | | " | 7 |
| Tobramycin | | | " | 7 |
| Amykacin | | | " | 7 |
| Opiate | | | " | 7 |
| Barbiturate | | | " | 7 |

1) siehe Seite 17/18

Tabelle 2 (Fortsetzung)

| Parameter | Verdünnungsfaktor | Meßzeit | Meßtechnik | Reaktions-/ Reagenzsegmente |
|---|---|---|---|---|
| Methadon | | | " | 7 |
| Amphetamin | | | " | 7 |
| Kokain | | | " | 7 |
| Morphin | | | " | 7 |
| Benzodiazepin | | | " | 7 |
| Propoxyphen | | | " | 7 |
| Theophyllin | | | " | 7 |
| Methotrexat | | | " | 7 |
| <u>Krebsindikatoren:</u> | | | | |
| $\alpha_1$-Foetoprotein | | | Analog T4 | 7 |
| Carcinoembryonales Antigen | | | " | 7 |

Die Erfindung ermöglicht auch die Bestimmung spezifischer Proteine, wie IgG, IgA, IgM, Transferin, $\alpha_1$-AT, $\alpha_2$-Makroglobulin, Haptoglobulin, β-Lipoprotein, $\alpha_1$-Glykoprotein und Albumin, wobei die TINIA-Meßtechnik angewendet wird und der Verdünnungsfaktor zweckmäßig 1 : 100 beträgt. Hierbei wird die Zunahme der Trübung in einer Lösung durch Bildung des Hapten-Antikörper-Komplexes gemessen. Das Verfahren ist dem Fachmann bekannt. Für diese Bestimmungen reicht ein Inkubationsschritt und demzufolge die Anordnung von zwei Reagenzsegmenten aus.

Die obgen Ausführungen zeigen, daß durch die Erfindung ein Analysensystem geschaffen wird, welches folgende Vorteile miteinander vereint: Es ist nur ein Minimum an Manipulationen von der Probeentnahme bis zum fertigen Analysenresultat erforderlich.

Es können sowohl eine Vielzahl einfacher, häufig eingesetzter Analysen auch spezielle komplizierte Einzelanalysen in gleicher Weise durchgeführt werden.

Die Menge des erforderlichen Probevolumens wird auf ein Minimum verringert mit entsprechender Minimalisierung von Reagenzbedarf und Reagenzkosten.

Die Durchführung kann einfach und mit wenig trainiertem Personal erfolgen.

Die Analysenfrequenz ist so hoch, wie bei den schnellsten bekannten automatisierten Analysensystemen.

Erfindungsgemäß ist es überraschenderweise auch möglich, das feste Reagenz in wesentlich geringeren Probeflüssigkeitsmengen aufzulösen und damit konzentriertere Lösungen zu gewinnen, als dies unter sonst vergleichbaren Verhältnissen möglich ist, wenn man Reagenzien von festen Trägern ohne Einfluß der Zentrifugalkraft ablöst.

Die Erfindung erlaubt weiter die Anwendung der verschiedenen denkbaren Meßmethoden, wie optische Durchstrahlung, Nephelometrie, Turbidimetrie, Reflexionsmessung, Fluoreszenzmessung, Lumineszenzmessung, radioaktive Strahlungsmessung, elektrische Messungen, wie Leitfähigkeit und dergleichen.

Die folgenden Beispiele erläutern die Erfindung weiter.

Beispiel 1

Dieses Beispiel beschreibt die Durchführung der Erfindung unter Verwendung eines Rotors, wie er in den Fig. 2 und 3 der Zeichnung dargestellt ist.

a) Meßvorgang
Einsatzelement

Zur Verwendung kommt ein Faservlies. Dieses besteht zu 40 % aus Polyamidfasern und 40 % aus Zellwolle. Es hat ein Aufnahmevermögen für wäßrige Lösungen von etwa 500 ml/m², bei einer Dicke von

0,5 mm und einem Flächengewicht von 75 g/m$^2$ (VS 532 der Fa. Binzer). Zur Beladung werden Tränklösungen von Reagenzien entsprechender Konzentration hergestellt. Diese werden entweder mit einer Pipette aufgeträufelt oder in einer Imprägnieranlage aufgebracht. Dabei wird das Papier in die Tränklösung getaucht, danach überschüssige Lösung zwischen zwei Rollen abgequetscht und im Luftstrom getrocknet. Die zur Beladung notwendigen Mengen ergeben sich aus der angestrebten Konzentration in der Küvette und den für die Reagenzien verschiedenen Elutionsgraden.

Die zugeschnittenen Reagenzpapiere (2 mm × 6 mm bis 6 mm × 6 mm) werden in die Schlitze 26 des Rotors (Fig. 2) gesteckt. Wegen der Kürze des Kanals beträgt die Breite 1 mm und es werden mindestens zwei Vliese nebeneinander gestellt, um ein Verrutschen der Vliese zu verhindern. Die Vliese werden mit Hilfe einer Pinzette eingesteckt und der abnehmbare Deckel zugleich Küvettenfenster, wieder aufgeschraubt. 18 µl verdünnter Probelösung werden in die Probekammer dosiert. Zentrifugation bei 2 880 rpm für 1 Sekunde bis 25 Sekunden. In diesem Schritt wird die verdünnte Probe in die ersten Vliese transportiert und löst das Reagenz auf.

Zentrifugation bei 12 000 rpm für 5 Sekunden. Hierdurch wird die Lösung aus dem Vlies in die Küvette getrieben. Auf dem Vlies bleiben nur minimale Mengen Lösung zurück. Anschließend wird gemischt.

Dieser Vorgang besteht aus 1 Sekunde Beschleunigung auf 12 000 rpm und 1 Sekunde Anhalten und wird 6- bis 20-mal wiederholt. Durch die Beschleunigungs- und Bremsschritte wird die Lösung in der Küvettenvorkammer gemischt. Diese Schüttelzyklen sind nicht notwendig, wenn nach dem letzten Vlies eine längere Wegstrecke zur Verfügung steht, auf der Mischkörper angeordnet sind. Anschließend wird 4 Sekunden lang bei 12 000 rpm zentrifugiert zum Austreiben von Luftblasen und Sedimentieren von z. B. Fasern aus dem Lichtweg heraus, bevor auf Meßgeschwindigkeit geschaltet wird. Die Messung erfolgt bei 2 880 rpm.

Diese Ausführungsform aber eignet sich insbesondere für die in Tabelle 1 angegebenen Bestimmungen.

b) Bestimmung von Glucose

Testzusammensetzung

100 mM Natriumphosphatpuffer, pH 7,0
0,77 mM 4-Aminoantipyrin
11 mM Phenol
$>$ 18 U/ml Glucoseoxidase
$>$ 1,1 U/ml Peroxidase

Belagung der Papiere (getränkt)

Enzym/Farbstoffvlies, 6 mm × 6 mm wird mit Lösung, enthaltend
1 680 mU Glucoseoxidase
210 mU Peroxidase
8,4 µg Aminoantipyrin
getränkt und getrocknet.

Puffervlies, 3 mm × 6 mm wird mit Lösung, enthaltend
228 µg Dinatriumhydrogenphosphat
312 µg Natriumdihydrogenphosphat
getränkt und getrocknet.

Der Kuppler, Phenol, wurde 11 mM mit der verdünnten Probe zugegeben.

Meßbedingungen :

Verdünnung 1 : 100 mit 0,2 % Triton-X-405.

Erste Zentrifugation bei 2 880 rpm für 5 Sekunden 6 mal mischen

Vliesanordnung :
1/2 Enzym/Farbstoff
Puffer
1/2 Enzym/Farbstoff

Messung der Absorption bei 500 nm.

Die Auswertung erfolgt nach der Pseudo-Endpunkt-Methode, d. h. durch Anpassung der Meßpunkte 25 bis 70 Sekunden an die Funktion nach einer bekannten mathematischen Methode.

Meßergebnisse :

Linearität :

Es wurden wäßrige Glucose-Standards unter Anwendung eines Rotationsphotometers vermessen. Die Ergebnisse sind in Fig. 8 graphisch dargestellt. Zwecks Methodenvergleichs wurden erfindungsgemäß ermittelte Werte mit manuell ermittelten Werten verglichen. In beiden Fällen wurde mit dem Standard 100 mg/dl geeicht. Die Ergebnisse zeigt Fig. 9, welche in graphischer Darstellung auf der Abszisse die manuell, auf der Ordinate die erfindungsgemäß ermittelten Werte angibt.

## Beispiel 2

Bestimmung von GOT (Aspartat-Aminotransferase)

Es wurde wie in Beispiel 1 beschrieben vorgegangen, unter Verwendung eines Einsatzelements gemäß Fig. 4 und 5.

1. Testzusammensetzung

Die Sollkonzentrationen wurden analog verschiedener Empfehlungen gewählt.

80 mM Tris, pH 7,8 bei 30 °C
240 mM L-Asparaginsäure
12 mM a-Ketoglutarsäure
2 000 U/l Malatdehydrogenase (MDH)
3 000 U/l Lactatdehydrogenase (LDH)
0,23 mM NADH.

Als Referenztest wurde der Monotest GOT nach IFCC, Best. Nr. 300667 Boehringer Mannheim verwendet.

2. Beladung der Papiere (getränkt)

Puffervlies, 3 mm × 6 mm, enthält
407 µg Trisbase
1 340 µg Asparaginsäure
pH 7,8 mit NaOH bei 30 °C.

Enzymvlies, 3 mm × 6 mm, enthält
84 mU MDH
126 mU LDH
76 µg Natriumketoglutarat
pH der Tränklösung war 7,4.

Puffervlies, 3 mm × 6 mm, enthält
6 µg Natrium-NADH
pH 9,95 mit 11 mM Natriumcarbonat.

3. Meßbedingungen

Verdünnung 1 + 10 mit 0,02 % Triton-X-405

Erste Zentrifugation bei 2 880 rpm für 1 Sekunde 6 mal mischen.

Vliesanordnung :
2/3 Enzym
Puffer
2/3 Farbstoff

Messung der Adsorption bei 340 nm.

4. Meßergebnisse

Präzision und Linearität :

Gemessen wurde am Rotationsphotometer mit 13 verschiedenen Verdünnungen des Kontrollserums (Percipath E von Boehringer Mannheim). Es wurden jeweils vier Konzentrationen in einem Rotor gemessen, wobei eine als Eichwert dient. Die Ergebnisse zeigt Fig. 10.

## Beispiel 3

Alkalische Phosphatase

Die Bestimmung erfolgte wie in Beispiel 2 mit folgender Testzusammensetzung :

Der Handtest als Bezugsmethode wird mit den hier aufgeführten Konzentrationen durchgeführt.

0,6 M D(−)-N-Methylglucamin
4 mM Magnesium-L-aspartat
12 mM Di-tris-para-nitrophenylphosphat
7,7 mM Tris, d. h. Trishydroxymethylaminomethan
0,2 % Triton X-405
pH 10,45 (bei 25 °C mit Salzsäure einstellen).

Beladung der Papiere (aufgeträufelt und getrocknet) :

Substratvlies, 3 mm × 6 mm
130 µg Tris-NPP
12 µg Tris

getrocknet wurde im Vakuum über Silikagel.

Puffervlies A, 4 mm × 6 mm
2 362 µg Methylglucamin
232 µg Methylglucaminhydrochlorid
58 µg Magnesiumaspartat.

Puffervlies B, 4 mm × 6 mm
3 836 µg Methylglucamin.

Meßbedingungen :

Verdünnung 1 + 40 mit 0,2 % Triton

Erste Zentrifugation bei 2 880 rpm für 25 s

20-mal Mischen.

Vliesanordnung : Puffer A Hilfsvlies
(Zentrum) (Küvette)
Puffer B Substrat
Messung der Absorption bei 410 nm.
Meßergebnisse :
Präzision :
Normbereich, 60 bis 170 U/l (opt.) Probe 100 U/l
$n = 13$
$\bar{A} = 33{,}2$ mE/min
VK = 8,7 %
Pathologischer Bereich, > 170 U/l : Probe 500 U/l
$n = 16$
$\bar{A} = 145{,}4$ mE/min
VK = 4,4 %

**Ansprüche**

1. Verfahren zur Durchführung analytischer Bestimmungen durch Mischen und Inkubieren einer Probelösung mit wenigstens einer Reagenzlösung und optische Messung eines Parameters im inkubierten Reaktionslösungsgemisch, wobei Mischen, Inkubieren und Messen während der Einwirkung einer Zentrifugalkraft durchgeführt werden, dadurch gekennzeichnet, daß man die Probelösung unter der Einwirkung der Zentrifugalkraft

a) mit einem löslichen Trockenreagenz zusammenbringt unter wenigstens teilweiser Auflösung desselben,

b) das Gemisch bzw. die Lösung durch eine Vielzahl sehr kleiner miteinander in Verbindung stehender Hohlräume schleudert,

wobei man Zentrifugalkraft und Strömungswiderstand der kleinen Hohlräume so aufeinander abstimmt, daß eine vollständige Auflösung und Mischung der Reaktionspartner und gegebenenfalls Inkubation eintritt, bevor die Reaktionslösung aus den kleinen Hohlräume in eine Meßkammer austritt, in der man die Messung vornimmt.

2. Verfahren nach Anspruch 1, dadurch gekennzeichnet, daß man nacheinander mehrere verschiedene Trockenreagenzien in der Probelösung auflöst.

3. Verfahren nach Anspruch 1 oder 2, dadurch gekennzeichnet, daß man bei gegebener Zentrifugalkraft die Strömungsgeschwindigkeit des Gemisches bzw. der Lösung verändert, indem man nacheinander kleine Hohlräume von unterschiedlichem mittleren Durchmesser durchströmen läßt.

4. Verfahren nach einem der vorhergehenden Ansprüche, dadurch gekennzeichnet, daß man kleine Hohlräume mit reaktiver Oberfläche, die mit wenigstens einem Bestandteil der Lösung bzw. des Gemisches in chemische Wechselwirkung tritt, durchströmt.

5. Verfahren nach Anspruch 4, dadurch gekennzeichnet, daß man reaktive Oberflächen der kleinen Hohlräume anwendet, welche Austauscheraktivität oder Affinitätschromatographie-Aktivität aufweisen.

6. Verfahren nach Anspruch 5, dadurch gekennzeichnet, daß die Oberfläche der kleinen Hohlräume enzymatisch oder immunologisch aktive Substanzen trägt.

7. Einsatzelement für einen Zentrifugalanalysenrotor, dadurch gekennzeichnet, daß es wenigstens ein lösliches Analysenreagenz in getrockneter Form enthält und eine Vielzahl sehr kleiner, miteinander in Verbindung stehender Hohlräume aufweist, welche eine Probeaufgabekammer (42) und eine Meßkammer (51) miteinander verbinden.

8. Einsatzelement nach Anspruch 7, dadurch gekennzeichnet, daß die Vielzahl sehr kleiner, miteinander in Verbindung stehender Hohlräume durch ein netzförmiges Element gebildet ist.

9. Einsatzelement nach Anspruch 8, dadurch gekennzeichnet, daß das netzförmige Element aus einem geflochtenen Netz besteht.

10. Einsatzelement nach Anspruch 8, dadurch gekennzeichnet, daß das netzförmige Element aus einem Vlies oder Papier besteht.

11. Einsatzelement nach Anspruch 8, dadurch gekennzeichnet, daß das netzförmige Element aus einem offenzelligen Schaumstoff besteht.

12. Einsatzelement nach einem der Ansprüche 7 bis 11, dadurch gekennzeichnet, daß die Vielzahl sehr kleiner, miteinander in Verbindung stehender Hohlräume durch eine strukturierte Oberfläche gebildet wird, welche mit einer glatten oder strukturierten Oberfläche abgedeckt ist.

13. Einsatzelement nach einem der Ansprüche 7 bis 12, dadurch gekennzeichnet, daß es Probeaufnahmekammer (42) oder/und Meßkammer (51) aufweist.

14. Einsatzelement nach einem der Ansprüche 7 bis 13, dadurch gekennzeichnet, daß es einen eine Vielzahl sehr kleiner, miteinander in Verbindung stehender Hohlräume aufweisenden länglichen Körper enthält, der mit einer Folie abgedichtet ist, die an wenigstens einer Schmalseite eine Schlaufe bildet, die eine Meßkammer (51) oder/und Probeaufgabekammer (42) definiert.

15. Einsatzelement nach einem der Ansprüche 7 bis 14, dadurch gekennzeichnet, daß es mehrere räumlich voneinander getrennt vorliegende Analysenreagenzien enthält.

16. Einsatzelement nach einem der Ansprüche 7 bis 15, dadurch gekennzeichnet, daß das Analysenreagenz als geformter Körper vorliegft.

17. Einsatzelement nach einem der Ansprüche 7 bis 16, dadurch gekennzeichnet, daß wenigstens ein Teil der Oberfläche der kleinen Hohlräume reaktiv ausgebildet ist und mit der Reaktionslösung in chemische Wechselwirkung treten kann.

18. Einsatzelement nach Anspruch 17, dadurch gekennzeichnet, daß an der Oberfläche enzymatisch oder immunologisch aktive Substanzen gebunden sind.

19. Einsatzelement nach Anspruch 17, dadurch gekennzeichnet, daß die Oberfläche reaktive Gruppen mit Ionenaustauschereigenschaften aufweist.

20. Einsatzelement nach einem der Ansprüche 7 bis 19, dadurch gekennzeichnet, daß zwischen Probeaufgabekammer (42) und Meßkammer (51) Abschnitte mit unterschiedlicher mittlerer Hohlraumgröße angeordnet sind, welche für eine durchströmende Flüssigkeit unterschiedlichen Strömungswiderstand aufweisen.

21. Einsatzelement nach einem der Ansprüche 7 bis 20, gekennzeichnet durch ein erstes Element, welches eine Vielzahl sehr kleiner, miteinander in Verbindung stehender Hohlräume aufweist und ein erstes Reagenz enthält, ein in Strömungsrichtung dahinter angeordnetes zweites Element mit einer Vielzahl sehr kleiner, miteinander in Verbindung stehender Hohlräume mit größerem Strömungswiderstand als das erste Element und ein in Strömungsrichtung dahinter angeordnetes drittes Element, welches eine Vielzahl sehr kleiner, miteinander in Verbindung stehender Hohlräume aufweist, die einen geringeren Strömungswiderstand besitzen als die Hohlräume des zweiten Elements, und ein zweites Reagenz enthält.

22. Einsatzelement nach Anspruch 21, dadurch gekennzeichnet, daß es zwischen dem zweiten und dem dritten Element ein weiteres Element mit einer Vielzahl sehr kleiner, miteinander in Verbindung stehender Hohlräume aufweist, welche eine reaktive Oberfläche aufweisen.

23. Einsatzelement nach einem der Ansprüche 7 bis 22, gekennzeichnet durch einen Abschnitt, in dem die sehr kleinen Hohlräume Molekularsiebstruktur aufweisen.

**Claims**

1. Process for the carrying out of analytical determinations by mixing and incubating of a sample solution with at least one reagent solution and optical measurement of one parameter in the incubated reaction solution mixture, whereby mixing, incubating and measuring are carried out during the action of a centrifugal force, characterised in that, under the influence of the centrifugal force

a) one brings the sample solution together with a soluble dry reagent, with at least partial dissolving thereof,

b) one centrifuges the mixture or the solution through a plurality of very small hollow spaces in connection with one another,

whereby one so coordinates centrifugal force and flow resistance of the small hollow spaces with one another that a complete dissolving and mixing of the reaction partners and possibly incubation takes place before the reaction solution passes from the small hollow spaces into a measuring chamber in which one carries out the measurement.

2. Process according to claim 1, characterised in that one successively dissolves several different dry reagents in the sample solution.

3. Process according to claim 1 or 2, characterised in that, at a given centrifugal force, one alters the rate of flow of the mixture or of the solution in that one allows flow through successive small hollow spaces of differing average diameter.

4. Process according to one of the preceding claims, characterised in that one allows flow through small hollow spaces with reactive surfaces which enter into chemical exchange action with at least one component of the solution or of the mixture.

5. Process according to claim 4, characterised in that one uses reactive surfaces of the small hollow spaces which have exchanger activity or an affinity chromatography activity.

6. Process according to claim 5, characterised in that the surface of the small hollow spaces carries enzymatically or immunologically active substances.

7. Insert element for a centrifugal analysis rotor, characterised in that it contains at least one soluble analysis reagent in dry form and has a plurality of very small hollow spaces in connection with one another which connect a sample providing chamber (42) with a measuring chamber (51) with one another.

8. Insert element according to claim 7, characterised in that the plurality of very small hollow spaces in connection with one another is formed by a mesh-shaped element.

9. Insert element according to claim 8, characterised in that the mesh-shaped element consists of an interwoven mesh.

10. Insert element according to claim 8, characterised in that the mesh-shaped element consists of a fleece or paper.

11. Insert element according to claim 8, characterised in that the mesh-shaped element consists of an open-celled foamed material.

12. Insert element according to one of claims 7 to 11, characterised in that the plurality of very small hollow spaces in connection with one another is formed by a structured surface, which is covered with a flat or structured surface.

13. Insert element according to one of claims 7 to 12, characterised in that it has sample reception chambers (42) and/or measuring chambers (51).

14. Insert element according to one of claims 4 to 13, characterised in that it contains a longitudinal body having a plurality of very small hollow spaces in connection with one another which body is sealed with a foil which, on at least one narrow side, forms a loop which defines a measuring chamber (51) and/or sample providing chamber (42).

15. Insert element according to one of claims 7 to 14, characterised in that it contains several analysis reagents present spatially separated from one another.

16. Insert element according to one of claims 7 to 15, characterised in that the analysis reagent is present as a formed body.

17. Insert element according to one of claims 7 to 16, characterised in that at least a part of the surface of the small hollow spaces is reactively formed and can enter into chemical exchange action with the reaction solution.

18. Insert element according to claim 17, characterised in that enzymatically or immunologically active substances are bound on to the surface.

19. Insert element according to claim 17, characterised in that the surface has reactive groups with ion exchange properties.

20. Insert element according to one of claims 7 to 19, characterised in that between sample supplying chamber (42) and measuring chamber (51) are arranged sections with different average hollow space size which have differing flow resistance for a throughflowing fluid.

21. Insert element according to one of claims 7 to 20, characterised by a first element, which has a plurality of very small hollow spaces in connection with one another and contains a first reagent, a second element, arranged therebehind in the direction of flow, with a plurality of very small hollow spaces in connection with one another with greater flow resistance than the first element and a third element, arranged therebehind in the direction of flow, which has a plurality of very small hollow spaces in connection with one another which possess a lower flow resistance than the hollow spaces of the second element and contains a second reagent.

22. Insert element according to claim 21, characterised in that, between the second and the third element, it has a further element with a plurality of very small hollow spaces in connection with one another which have a reactive surface.

23. Insert element according to one of claims 7 to 22, characterised by a section in which the very small hollow spaces have a molecular sieve structure.

**Revendications**

1. Procédé d'exécution de déterminations analytiques par mélange et incubation d'une solution d'échantillon avec au moins une solution de réactif et mesure optique d'un paramètre dans le mélange de solution réactionnelle incubé, le mélange, l'incubation et la mesure étant effectués pendant l'action d'une force centrifuge, caractérisé en ce que sous l'action de la force centrifuge,

a) on met en présence la solution d'échantillon avec un réactif sec soluble avec dissolution au moins partielle de celui-ci,

b) on centrifuge le mélange ou la solution à travers un grand nombre de très petites cavités reliées entre elles,

en ajustant la force centrifuge et la résistance à l'écoulement des petites cavités l'une à l'autre de telle sorte qu'il se produise une dissolution et un mélange complets des partenaires de la réaction et le cas échéant une incubation, avant que la solution réactionnelle ne sorte des petites cavités dans une chambre de mesure dans laquelle on effectue la mesure.

2. Procédé suivant la revendication 1, caractérisé en ce qu'on dissout successivement plusieurs réactifs secs différents dans la solution d'échantillon.

3. Procédé suivant la revendication 1 ou 2, caractérisé en ce qu'on modifie, pour une force centrifuge donnée, la vitesse d'écoulement du mélange ou de la solution en faisant traverser successivement de petites cavités de diamètres moyens différents.

4. Procédé suivant l'une des revendications précédentes, caractérisé en ce qu'on fait passer à travers de petites cavités ayant une surface réactive qui entre dans une interaction chimique avec au moins un constituant de la solution ou du mélange.

5. Procédé suivant la revendication 4, caractérisé en ce qu'on utilise des surfaces réactives des petites cavités qui présentent une activité d'échange ou une activité de chromatographie d'affinité.

6. Procédé suivant la revendication 5, caractérisé en ce que la surface des petites cavités porte des substances enzymatiquement ou immunologiquement actives.

7. Elément d'insertion pour un rotor d'analyse par centrifugation, caractérisé en ce qu'il présente au moins un réactif d'analyse soluble sous forme séchée et un grand nombre de cavités très petites, en liaison les unes avec les autres, qui relient entre elles une chambre de chargement des échantillons (42) et une chambre de mesure (51).

8. Elément d'insertion suivant la revendication 7, caractérisé en ce que le grand nombre de cavités très petites, en liaison les unes avec les autres est formé par un élément en forme de réseau.

9. Elément d'insertion suivant la revendication 8, caractérisé en ce que l'élément en forme de réseau est constitué d'un réseau tressé.

10. Elément d'insertion suivant la revendication 8, caractérisé en ce que l'élément en forme de réseau est constitué d'un non-tissé ou d'un papier.

11. Elément d'insertion suivant la revendication 8, caractérisé en ce que l'élément en forme de réseau est constitué d'une mousse plastique à cellules ouvertes.

12. Elément d'insertion suivant l'une des revendications 7 à 11, caractérisé en ce que le grand nombre de cavités très petites, en relation les unes avec les autres est formé par une surface structurée qui est recouverte d'une surface lisse ou structurée.

13. Elément d'insertion suivant l'une des revendications 7 à 12, caractérisé en ce qu'il présente une chambre de chargement des échantillons (42) ou/et une chambre de mesure (51).

14. Elément d'insertion suivant l'une des revendications 7 à 13, caractérisé en ce qu'il contient un corps allongé contenant un grand nombre de cavités très petites, en liaison les unes avec les autres, qui est recouvert d'une feuille qui forme sur au moins un petit côté un nœud coulant qui définit une chambre de mesure (51) ou/et une chambre de chargement de l'échantillon (42).

15. Elément d'insertion suivant l'une des revendications 7 à 14, caractérisé en ce qu'il contient plusieurs réactifs d'analyse se trouvant séparés spatialement les uns des autres.

16. Elément d'insertion suivant l'une des revendications 7 à 15, caractérisé en ce que le réactif d'analyse se trouve sous forme de corps moulé.

17. Elément d'insertion suivant l'une des revendications 7 à 16, caractérisé en ce qu'au moins une partie de la surface des petites cavités est rendue réactive et peut entrer dans une interaction chimique avec la solution réactionnelle.

18. Elément d'insertion suivant la revendication 17, caractérisé en ce que des substances enzymatiquement ou immunologiquement actives sont liées à la surface.

19. Elément d'insertion suivant la revendication 17, caractérisé en ce que la surface présente des groupes réactifs avec des propriétés d'échange d'ions.

20. Elément d'insertion suivant l'une des revendications 7 à 19, caractérisé en ce qu'entre la chambre de chargement de l'échantillon (42) et la chambre de mesure (51) sont disposés des tronçons ayant une grandeur de cavité moyenne différente, qui présentent une résistance à l'écoulement différente pour un liquide les traversant.

21. Elément d'insertion suivant l'une des revendications 7 à 20, caractérisé par un premier élément qui présente un grand nombre de cavités très petites, en liaison les unes avec les autres, et contient un premier réactif, un second élément disposé derrière celui-ci dans la direction de l'écoulement avec un grand nombre de cavités très petites, en liaison les unes avec les autres ayant une résistance à l'écoulement plus élevée que le premier élément et un troisième élément, disposé derrière celui-ci dans la direction de l'écoulement, qui présente un grand nombre de cavités très petites, en liaison les unes avec les autres, qui possèdent une résistance à l'écoulement plus faible que les cavités du second élément, et contient un second réactif.

22. Elément d'insertion suivant la revendication 21, caractérisé en ce qu'il présente entre le second et le troisième élément un autre élément avec un grand nombre de cavités très petites, en liaison les unes avec les autres, qui présentent une surface réactive.

23. Elément d'insertion suivant l'une des revendications 7 à 22, caractérisé par un tronçon dans lequel les très petites cavités présentent une structure de tamis moléculaire.

FIG.1

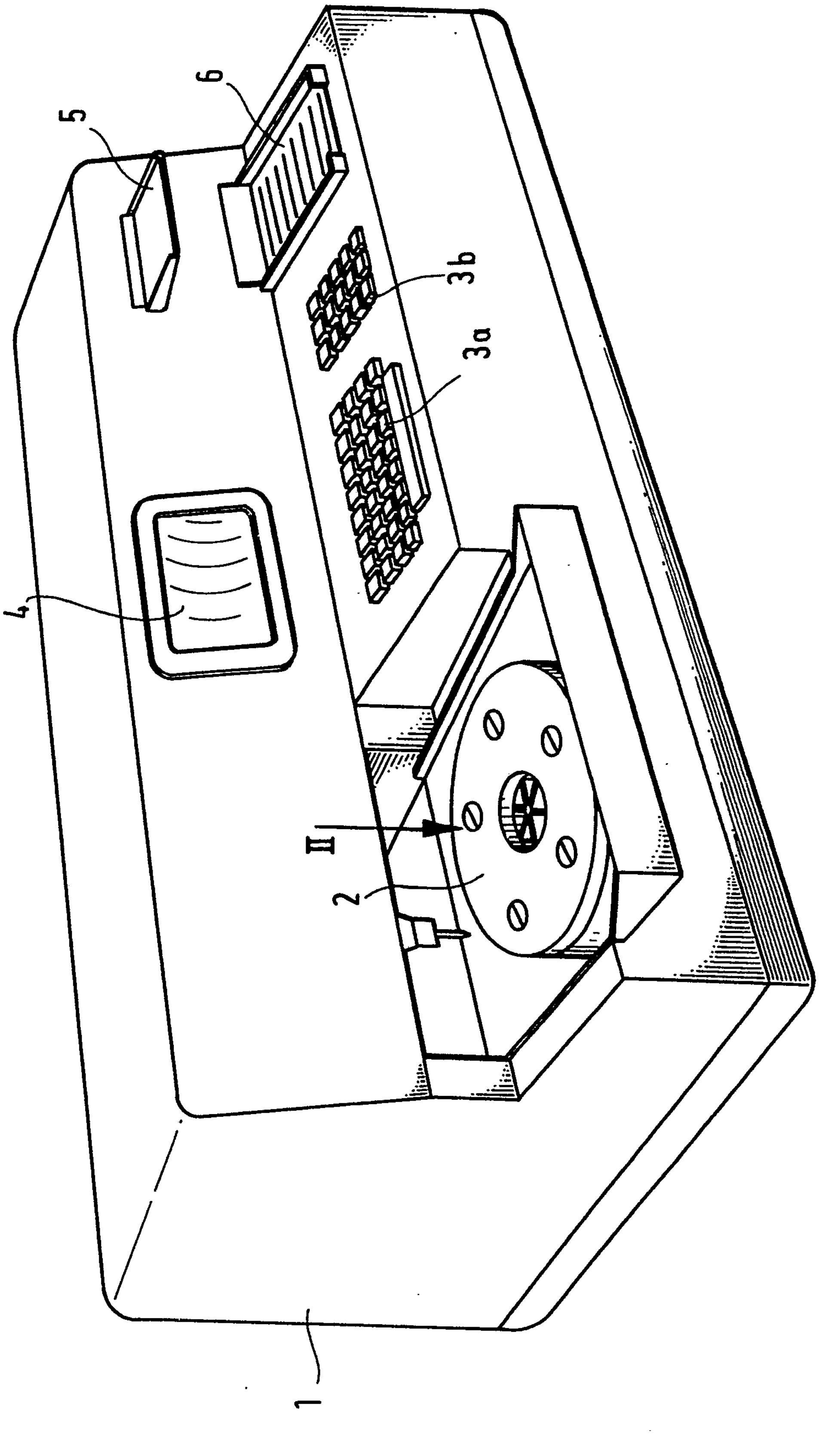

FIG. 2

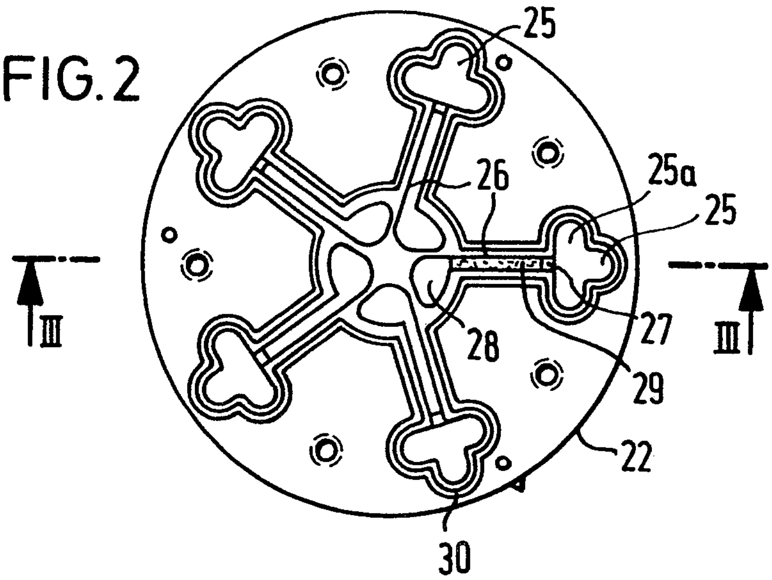


FIG. 3

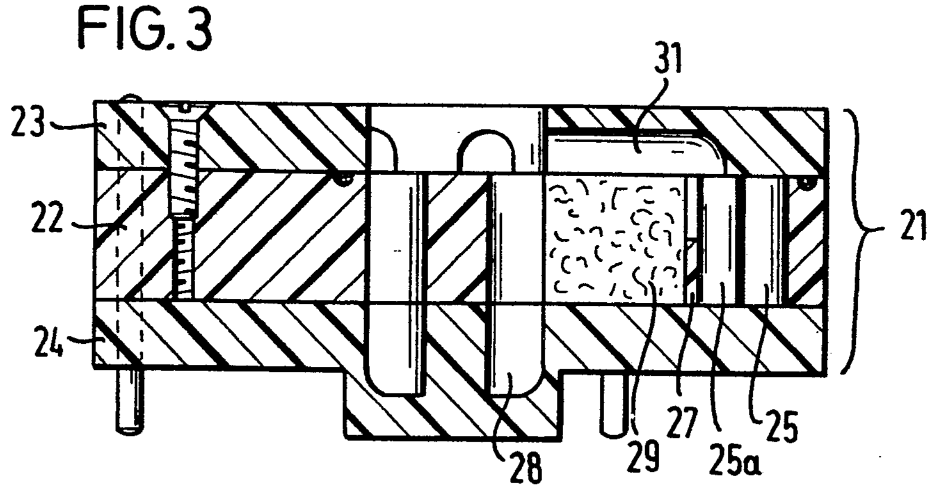


FIG. 6

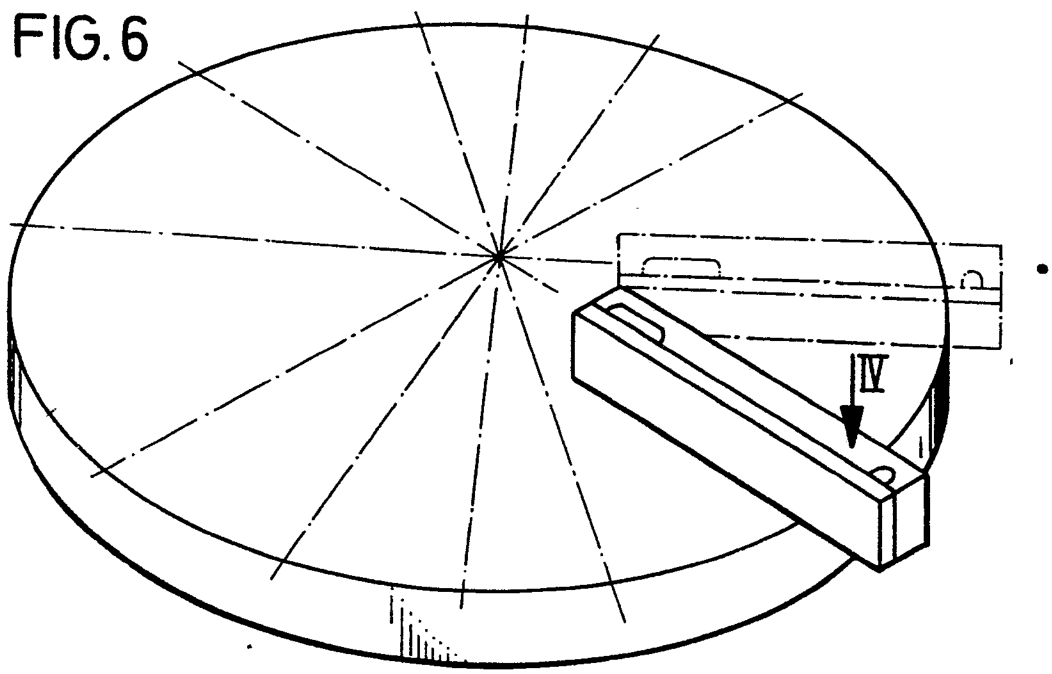

FIG. 4

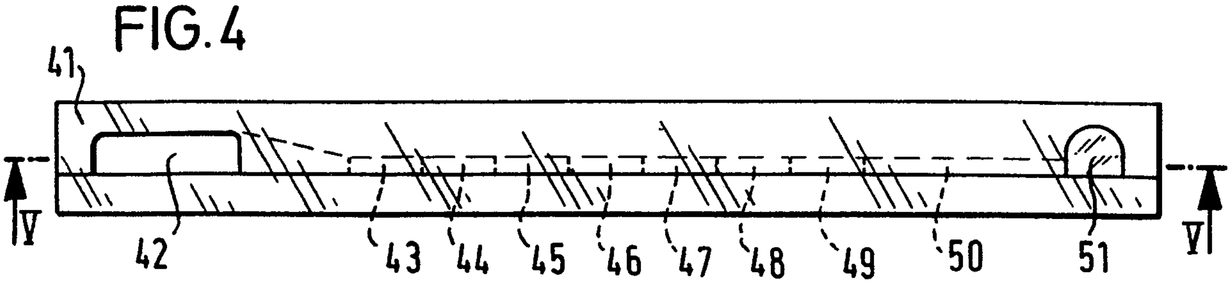


FIG. 5

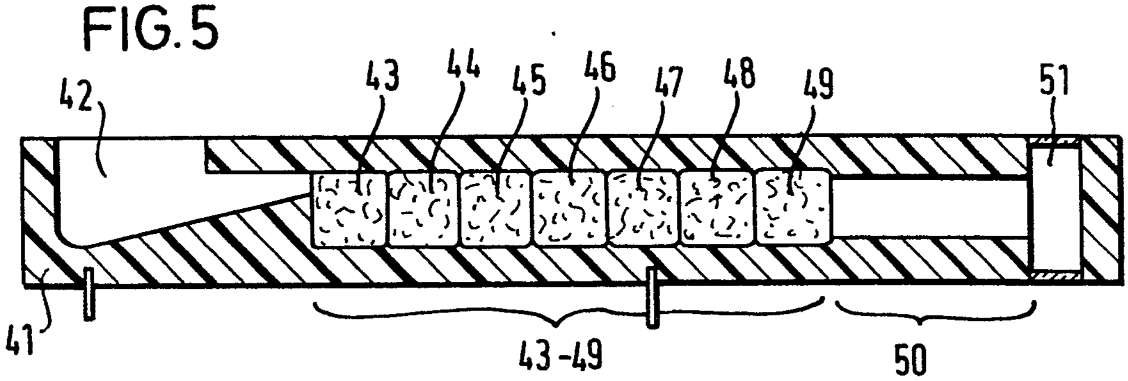


FIG. 7a

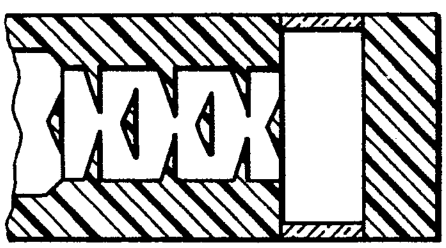

FIG. 7b

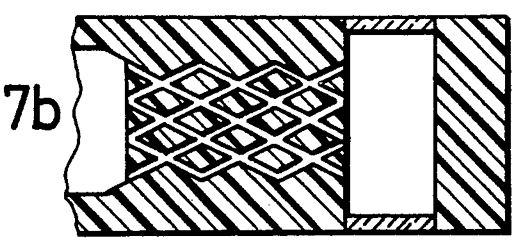

FIG. 7c

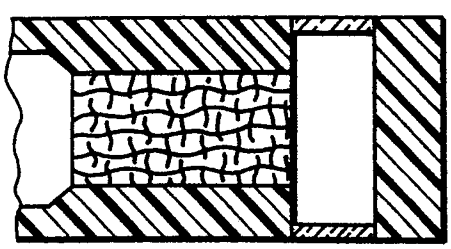

FIG. 8

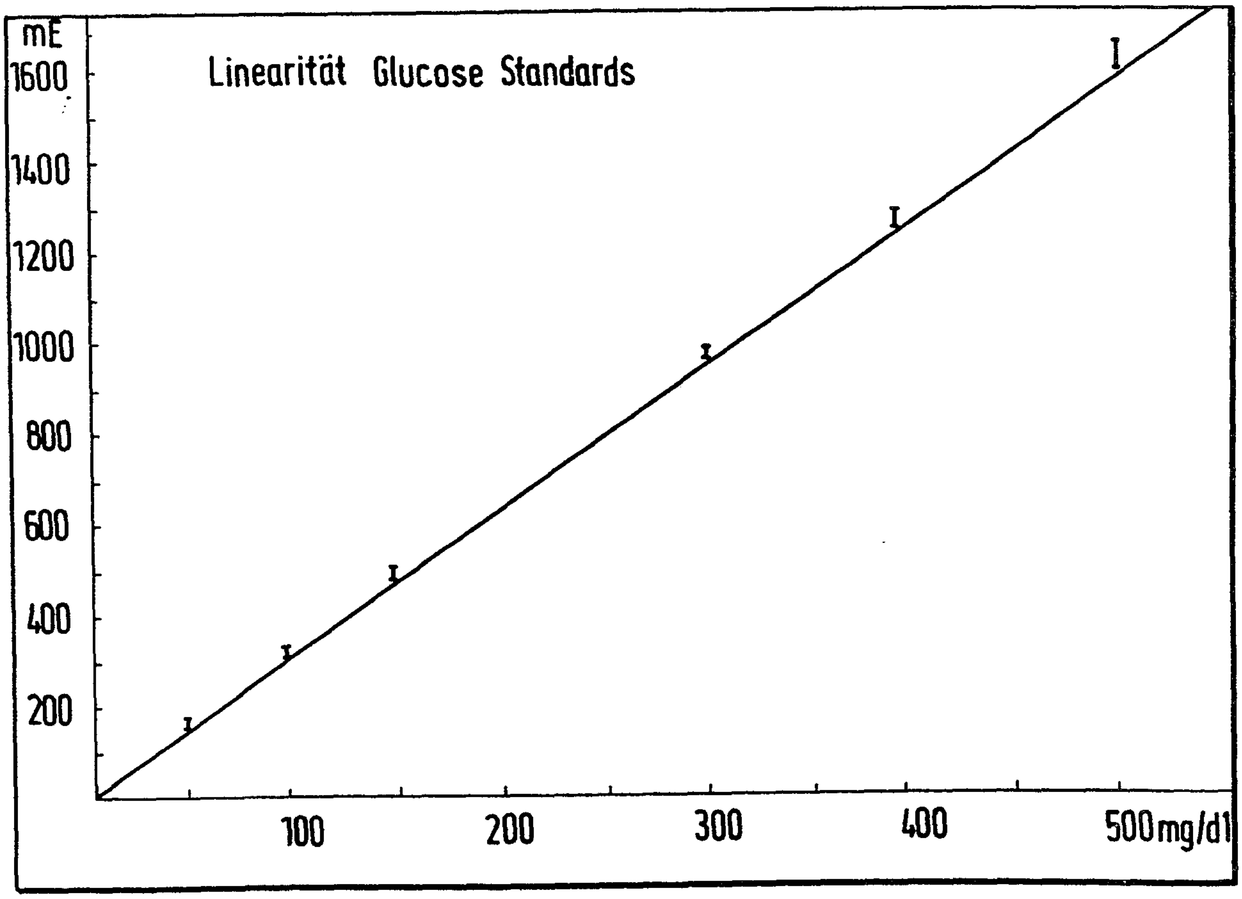
mE
1600
1400
1200
1000
800
600
400
200
Linearität Glucose Standards
100
200
300
400
500 mg/dl

FIG. 9

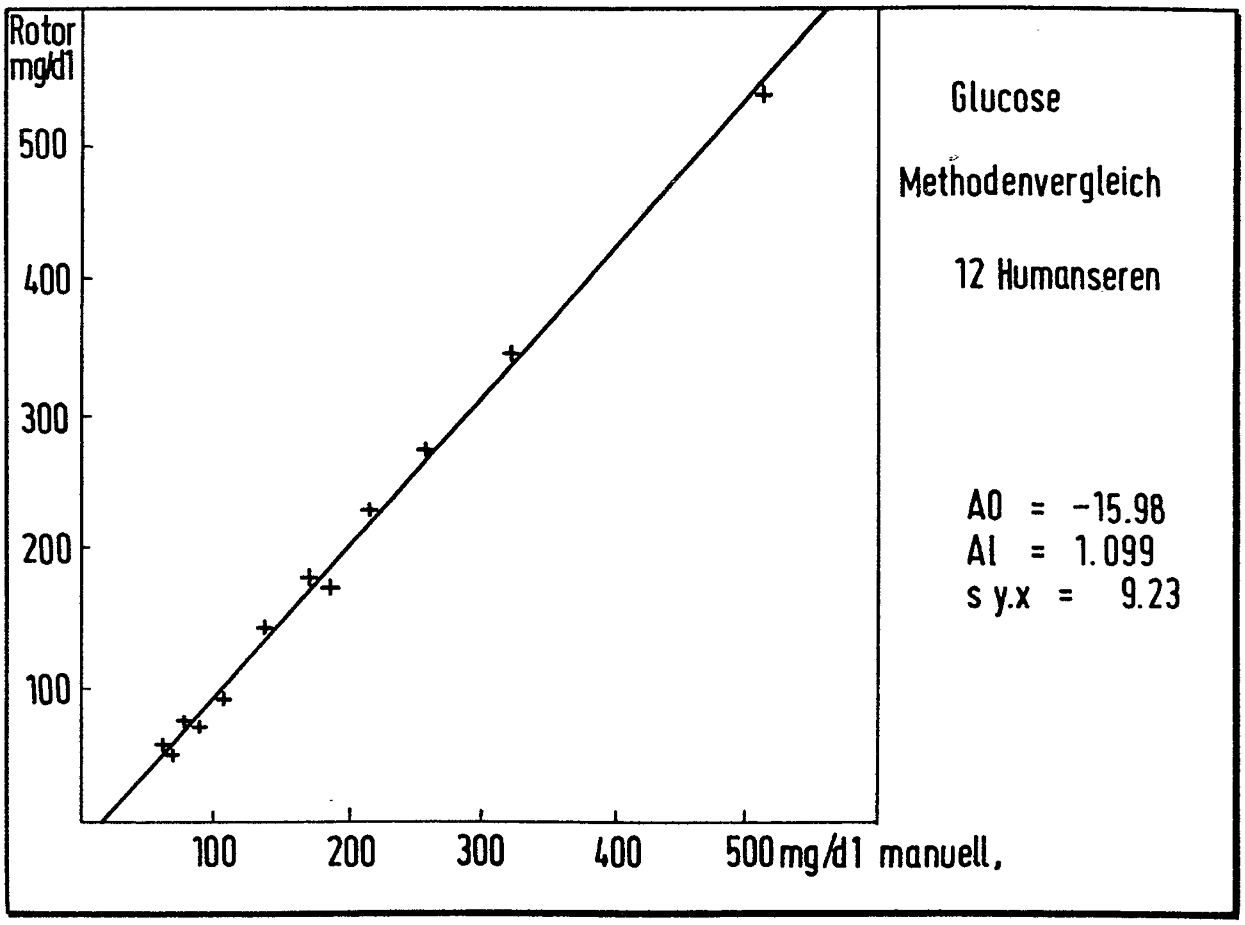
Rotor
mg/dl
500
400
300
200
100
100
200
300
400
500 mg/dl manuell,
Glucose
Methodenvergleich
12 Humanseren
A0 = -15.98
A1 = 1.099
s y.x = 9.23

FIG. 10

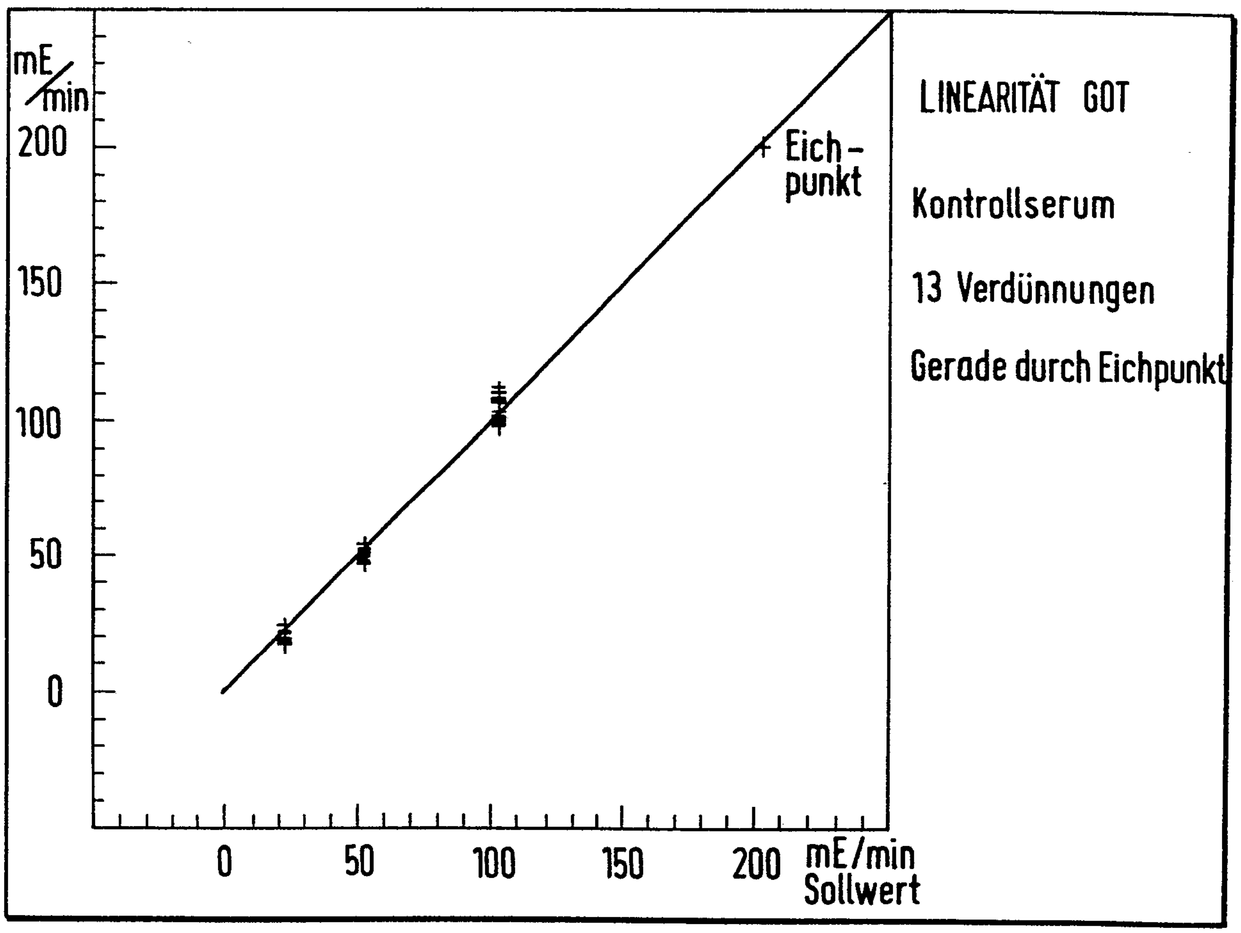
mE/min
200
150
100
50
0
Eich-punkt
0
50
100
150
200
mE/min Sollwert
LINEARITÄT GOT
Kontrollserum
13 Verdünnungen
Gerade durch Eichpunkt